# EUROPEAN PATENT APPLICATION

(11) **EP 4 549 948 A1**
(43) Date of publication of application: **07.05.2025**
(21) Application number: 24209808.5
(22) Date of filing: 30.10.2024
(51) Int. Cl.: G01N 33/72, A61K 9/00, A61K 38/42, C07K 14/805

(54) **METHOD OF PRODUCING SOLUTION CONTAINING HEMOGLOBIN A2, METHOD OF PRODUCING SOLUTION CONTAINING HEMOGLOBIN A2 AND HEMOGLOBIN F, AND METHOD OF PRODUCING HEMOGLOBIN PRECISION CONTROL SUBSTANCE**

(30) Priority: 30.10.2023 JP 2023185917
(71) Applicant: ARKRAY, Inc., Kyoto-shi, Kyoto 601-8045 (JP)
(72) Inventor: AMANO, Chie, Kyoto, 602-0008 (JP); OMOTO, Kazumasa, Kyoto, 602-0008 (JP)
(74) Representative: Dehns

(57) **Abstract**

A method of producing a solution containing hemoglobin A2 and a method of producing a hemoglobin precision control substance, the methods including contacting a solution containing hemoglobin and having a pH of from 7.8 to 9.5 with an anion exchanger, are provided.

## Description

### BACKGROUND

### Technical Field

The present disclosure relates to a method of producing a solution containing hemoglobin A2, a method of producing a solution containing hemoglobin A2 and hemoglobin F, and a method of producing a hemoglobin precision control substance.

### Related Art

In the production of precision control substances used as references in quantitative determination of hemoglobin (hereinafter, also referred to as "Hb"), human blood is generally used as a raw material. Hb in healthy adult human blood contains about from 95 to 96% of HbA, about from 2 to 3% of HbA2, and about 0.5 to 1.5% of HbF.

However, since precision control substances are used to prepare samples having Hb concentrations of higher than clinical decision limits as well as samples having Hb concentrations of equal to or less than clinical decision limits, Hb concentrations of precision control substances are in some cases set to be higher than the Hb concentrations in healthy adult human blood. For example, since HbF is present in umbilical cord blood in a proportion of about 85% with respect to the total Hb, umbilical cord blood is generally used to prepare HbF control samples. Japanese Patent Application Laid-Open (JP-A) No. 2016-075504 describes an HbF control sample containing sucrose and a mixed hemolysate of a hemolysate of human red blood cells and human umbilical cord blood.

### SUMMARY OF THE INVENTION

### Technical Problem

Blood from β-thalassemia patients contains a high proportion of HbA2, and therefore, the blood from β-thalassemia patients are in some cases used as a raw material in the production of control samples of HbA2. However, since the blood from β-thalassemia patients is rarely available, it is a challenge to address how to secure solutions with high proportions of HbA2.

In view of such circumstances, the present disclosure is directed to a method of producing a solution containing HbA2, a method of producing a solution containing HbA2 and HbF, and a method of producing an Hb precision control substance, in which a solution having a high proportion of HbA2 can be obtained from readily available blood samples.

### Solution to Problem

Means for solving the foregoing problem include the following aspects.
(1) A method of producing a solution containing hemoglobin A2, the method including contacting a solution containing hemoglobin and having a pH of from 7.8 to 9.5 with an anion exchanger.
(2) The method of producing a solution containing hemoglobin A2 according to (1), further including recovering the solution after the contacting with the anion exchanger.
(3) The method of producing a solution containing hemoglobin A2 according to (1) or (2), wherein the solution containing hemoglobin has a pH of from 8.0 to 9.0.
(4) The method of producing a solution containing hemoglobin A2 according to any one of (1) to (3), wherein the solution after the contacting with the anion exchanger has a higher proportion of hemoglobin A2 with respect to a total amount of hemoglobin as compared with the solution before the contacting with the anion exchanger.
(5) The method of producing a solution containing hemoglobin A2 according to any one of (1) to (4), wherein the solution containing hemoglobin and having a pH of from 7.8 to 9.5 contains a buffer solution and hemoglobin derived from blood of a human subject that has no hemoglobin abnormality.
(6) A method of producing a solution containing hemoglobin A2 and hemoglobin F, the method including mixing a solution containing hemoglobin A2, obtained by the production method according to any one of (1) to (5), and a solution containing hemoglobin F.
(7) A method of producing a hemoglobin precision control substance, the method including contacting a solution containing hemoglobin and having a pH of from 7.8 to 9.5 with an anion exchanger.
(8) The method of producing a hemoglobin precision control substance according to (7), further including recovering the solution after the contacting with the anion exchanger.
(9) The method of producing a hemoglobin precision control substance according to (7) or (8), wherein the solution containing hemoglobin has a pH of from 8.0 to 9.0.
(10) The method of producing a hemoglobin precision control substance according to any one of (7) to (9), wherein the solution after the contacting with the anion exchanger has a higher proportion of hemoglobin A2 with respect to a total amount of hemoglobin as compared with the solution before the contacting with the anion exchanger.
(11) The method of producing a hemoglobin precision control substance according to any one of (7) to (10), wherein the solution containing hemoglobin and having a pH of from 7.8 to 9.5 contains a buffer solution and hemoglobin derived from blood of a human subject that has no hemoglobin abnormality.
(12) The method of producing a hemoglobin precision control substance according to any one of (7) to (11), the method including mixing the solution obtained by the contacting with the anion exchanger, and a solution containing hemoglobin F.

### Advantageous Effects of Invention

The present disclosure provides a method of producing a solution containing HbA2, a method of producing a solution containing HbA2 and HbF, and a method of producing an Hb precision control substance, in which a solution having a high proportion of HbA2 can be obtained from readily available blood samples.

### DETAILED DESCRIPTION OF THE INVENTION

Hereinafter, embodiments of the present disclosure will be described in detail. However, embodiments of the present disclosure are not limited to the following embodiments. Components (including element steps and the like) in the following embodiments are not essential unless otherwise specified. The same applies to numerical values and their ranges, and the numerical values and their ranges do not limit the embodiments of the present disclosure.

In the present disclosure, an element described in a singular form does not eliminate the presence of plural elements as long as there is no technical contradiction, unless otherwise specified.

In the present disclosure, the term "step" includes not only a step independent of other steps but also a step that cannot be clearly distinguished from other steps as long as the purpose of the step is achieved.

In the present disclosure, a numerical range indicated using "(from) ... to" includes the numerical values described before and after "to" as the minimum value and the maximum value, respectively.

In numerical ranges described herein in a stepwise manner, the upper limit value or the lower limit value of one numerical range may be replaced with the upper limit value or the lower limit value of another numerical range described in a stepwise manner. Further, in numerical ranges described herein, the upper limit value or the lower limit value of a numerical range may be replaced with a value described in the Examples section.

In the present disclosure, the total amount of Hb in a solution is measured by a cyanmethemoglobin method. Specifically, for example, the total amount of Hb can be measured with HEMOGLOBIN TEST WAKO (FUJIFILM Wako Pure Chemical Corporation).

In the present disclosure, the proportions of HbA2 and HbF in Hb are measured based on the peak area ratios obtained by high-performance liquid chromatography. The high-performance liquid chromatography is performed under the following conditions or equivalent conditions thereto. The equivalent conditions refer to conditions by which the elution ability of Hb and respective Hb types is not substantially influenced (for example, the error in the measurement of each of the proportions of HbA2 and HbF is ± 0.5% or less).

As an apparatus, ADAMS HA-8180T (manufactured by ARKRAY Inc.) is used. COLUMN UNIT 80T (manufactured by ARKRAY Inc.), which is filled with 0.45 ml of a hydrophilic polymer composed of a methacrylic acid ester copolymer as a filler, is used for the apparatus. As reagents, 80A as an eluent A, 80B as an eluent B, and 80CT as an eluent C (manufactured by ARKRAY Inc.) are used for the elution of Hb from the column, and 80H is used as a hemolysis/cleaning solution. The composition and pH of each of 80A, 80B, 80CT, and 80H are as follows. The reagents are allowed to flow in the column at a flow rate of 1.7 ml/min.
Eluent A (80A): phosphate buffer solution (pH 5.35 ± 0.05)
Eluent B (80B): phosphate buffer solution (pH 8.05 ± 0.20)
Eluent C (80CT): phosphate buffer solution (pH 7.0 to 7.4)
Hemolysis/cleaning solution (80H): phosphate buffer solution containing a surfactant (pH 7.5 ± 0.1)

### <Method of Producing Solution Containing HbA2>

A method of producing a solution containing HbA2 in an embodiment of the present disclosure includes contacting a solution containing Hb and having a pH of from 7.8 to 9.5 with an anion exchanger (hereinafter, also referred to as "contacting step"). Hereinafter, the solution containing Hb before the contacting with the anion exchanger is also referred to as "Hb solution", and the solution containing HbA2 obtained after the contacting with the anion exchanger is also referred to as "HbA2 solution". The resulting HbA2 solution may contain components other than HbA2 (e.g., other hemoglobins such as HbA0, HbA1, and HbF, a buffer, a stabilizer, etc.).

The inventors have found that, when an Hb solution obtained from healty adult human blood is purified by an anion exchanger, the resulting solution has a high proportion of HbA2 with respect to the total Hb at a certain pH. This phenomenon is described below in detail. The isoelectric point of Hb is about 7, and therefore, a high pH of the Hb solution leads to anion formation of the Hb and an increase in the binding ability of the anion exchanger to the Hb. In other words, as the pH of the Hb solution increases, the amount of the Hb unbound to the anion exchanger and remaining in the solution is decreased. Similarly, the amount of HbA2 remaining in the solution is also believed to be decreased as the pH increases. However, it has been found that, when the Hb solution has a specific pH, the proportion of the HbA2 unbound to the anion exchanger and remaining in the solution with respect to the total Hb is increased as the pH increases. This is believed to be because, when the Hb solution has a pH within a specific range, hemoglobins other than HbA2 are preferentially bound to the anion exchanger, and the HbA2 concentration is relatively increased. However, embodiments of the present disclosure are not limited by the presumptive mechanism.

By the method of producing an HbA2 solution according to the present disclosure, an HbA2 solution having a high proportion of HbA2 can be prepared from healthy adult human blood, for example. Therefore, it is not necessary to use blood having a high proportion of HbA2 (e.g., blood from β-thalassemia patients). In the method of producing HbA2 according to the present disclosure, the HbA2 solution may be prepared from blood having a high proportion of HbA2 (e.g., blood from β-thalassemia patients), or from blood having a proportion of HbA2 within or below a normal range.

The solution after the contacting with the anion exchanger typically has a higher proportion of HbA2 with respect to the total amount of Hb as compared with the Hb solution before the contacting with the anion exchanger.

### [Contacting Step]

In the contacting step, an Hb solution having a pH of from 7.8 to 9.5 is contacted with an anion exchanger. The origin of the Hb solution is not particularly limited, and examples thereof include human or non-human animal blood, and human blood is preferable. Examples of the non-human animal include a non-human mammal (monkey, dog, cat, mouse, rat, rabbit, cow, horse, pig, sheep etc.) and a bird (chicken, quail etc.). An Hb solution, obtained by extracting Hb from human or non-human animal blood and optionally subjecting the Hb to a purification treatment, is preferably used. From the viewpoint of availability, the blood is preferably peripheral blood.

For example, the Hb solution may be prepared by hemolyzing human or non-human animal blood to extract the Hb. The hemolysis may be performed by, for example, adding a buffer solution to a precipitate obtained by centrifugation of whole blood obtained by blood drawing, and freezing and thawing the resultant. The Hb solution may also be obtained by centrifuging the solution obtained by the hemolysis as described above, and recovering the supernatant. The Hb solution may also be obtained by further purifying the supernatant recovered as described above by dialysis or the like.

The concentration of the Hb in the Hb solution to be contacted with the anion exchanger is preferably from 28 to 41 g/L, more preferably from 32 to 37 g/L, and still more preferably from 34 to 35 g/L.

The proportion of HbA2 with respect to the total Hb in the Hb solution to be contacted with the anion exchanger is preferably 1.0% or more, more preferably 1.5% or more, and still more preferably 2.0% or more, from the viewpoint of efficient condensation of HbA2. The proportion may be 5.0% or less, may be 4.0% or less, or may be 3.0% or less. Accordingly, the proportion may be from 1.0 to 5.0%, may be from 1.5 to 4.0%, or may be from 2.0 to 3.0%.

The Hb solution preferably contains a buffer solution for pH adjustment. Examples of the buffer solution include a Tris buffer solution (Tris-HCl etc.), a carbonate buffer solution, a HEPES buffer solution, and a phosphate buffer solution.

The pH of the Hb solution to be contacted with the anion exchanger is from 7.8 to 9.5. From the viewpoint of suppressing the binding of the HbA2 to the anion exchanger and increasing the amount of the HbA2 to be recovered, the pH of the Hb solution is preferably 9.0 or less. From the viewpoint of increasing the proportion of the HbA2 in the resulting HbA2 solution, the pH of the Hb solution is preferably 8.0 or more, more preferably 8.3 or more, still more preferably 8.5 or more, and particularly preferably 8.8 or more. From these viewpoints, the pH of the Hb solution is preferably from 8.0 to 9.0, more preferably from 8.3 to 9.0, still more preferably from 8.5 to 9.0, and particularly preferably from 8.8 to 9.0. In the present disclosure, the pH refers to the pH at 25°C.

In an aspect, the Hb solution contains a buffer solution and Hb derived from blood of a human subject that has no Hb abnormality. Here, the "Hb abnormality" includes abnormality owing to a large or small amount of Hb as compared with healthy human subject blood, abnormality owing to a high or low proportion of HbA2 in the total Hb as compared with healthy human subject blood, and any other structural and/or functional abnormality of Hb, and preferably refers to abnormality owing to a high or low proportion of HbA2 in the total Hb as compared with healthy human subject blood. The abnormality in the amount of Hb as compared with healthy human subject blood can be determined by whether or not the Hb concentration in the blood deviates from a predetermined normal range. Similarly, the abnormality in the proportion of HbA2 in the total Hb as compared with healthy human subject blood can be determined by whether or not the proportion of HbA2 deviates from a predetermined normal range. Examples of the buffer solution include the buffer solutions described above.

Examples of the anion exchanger include a strongly basic anion exchange resin and a weakly basic anion exchange resin. Examples of the strongly basic anion exchange resin include an anion exchange resin having, as an ion exchange group, a quaternary amine exchange group (trimethylammonium group, diethanolammonium group, etc.). Examples of the weakly basic anion exchange resin include an anion exchange resin having, as an ion exchange group, a tertiary amine exchange group (polyamine, dimethylamine, etc.), a secondary amine exchange group, primary amine exchange group, or the like. From the viewpoint that the ion exchange can be performed within a broad pH range, a strongly basic anion exchange resin is preferable, and an anion exchange resin having a quaternary amine exchange group is more preferable. For the anion exchange resin, a commercially available product may be used. Examples of the commercially available product include Macro-PrepHighQ (Bio-Rad Laboratories, Inc.).

The manner of contacting the Hb solution with the anion exchanger is not particularly limited. For example, the Hb solution and the anion exchanger may be mixed, or the Hb solution may be loaded to a column filled with the anion exchanger, and the former is simple.

### [Other Steps]

The method of producing an HbA2 solution according to the present disclosure may include other steps, in addition to the contacting step.

For example, the method of producing an HbA2 solution may include recovering a solution after the contacting with the anion exchanger. In other words, the method of producing an HbA2 solution may include separating the HbA2 solution after the contacting with the anion exchanger from the anion exchanger, and recovering the HbA2 solution. As described above, when the solution containing Hb and having a pH of from 7.8 to 9.5 is contacted with the anion exchanger, the proportion of HbA2 with respect to the total amount of Hb in the solution is relatively increased, and therefore, a solution having a high concentration of HbA2 can be selectively obtained by recovering the solution.

As the recovery method, it is simple to use a method in which the anion exchanger is precipitated by centrifugation and the supernatant is recovered. In a case in which the Hb solution is loaded to a column filled with the anion exchanger, the flow-through may be recovered.

The method of producing an HbA2 solution may further include, after contacting the Hb solution with the anion exchanger to recover the HbA2 solution, cleaning the anion exchanger with a buffer solution and thereafter recovering the supernatant (or flow-through). By the cleaning process, HbA2 that has not been able to be recovered by a single recovery of the HbA2 solution can be recovered, and the amount of recovery can be increased. The number of times of the cleaning may be once, twice or more, and may be, for example, from 1 to 10 times, from 1 to 5 times, or from 1 to 3 times.

The method of producing an HbA2 solution may further include contacting the recovered HbA2 solution with the anion exchanger again, and then recovering the solution. In other words, the contacting of the solution with the anion exchanger may be repeated.

The method of producing an HbA2 solution may further include enriching the HbA2 solution obtained by contacting the Hb solution with the anion exchanger. The enrichment may be performed by, for example, precipitating the protein in the HbA2 solution with ammonium sulfate, recovering the precipitate by centrifugation, and adding a buffer solution.

The method of producing an HbA2 solution may include further purifying the HbA2 solution obtained by contacting the Hb solution with the anion exchanger. The purification may be performed after the step of enriching the HbA2 solution. The purification can be performed by dialysis or the like.

### <Method of Producing Solution Containing HbA2 and HbF>

A method of producing a solution containing HbA2 and HbF in an embodiment of the present disclosure includes mixing the HbA2 solution, obtained by the method of producing an HbA2 solution, and a solution containing HbF. Hereinafter, the solution containing HbF before the mixing is also referred to as "HbF solution", and the resulting solution containing HbA2 and HbF is also referred to as "HbA2/HbF solution". In this embodiment, for example, a precision control substance containing HbA2 and HbF can be produced. The resulting HbA2/HbF solution may contain components other than HbA2 and HbF (e.g., other hemoglobins such as HbA0 and HbA1, a buffer, a stabilizer etc.).

The origin of the HbF solution is not particularly limited, and examples thereof include human or non-human animal blood, and human blood is preferable. Examples of the non-human animal are as described above. An HbF solution, obtained by extracting Hb from human or non-human animal blood and optionally subjecting the HbF to a purification treatment, is preferably used. From the viewpoint of containing a large amount of HbF, the blood is preferably umbilical cord blood.

For example, the HbF solution may be prepared by hemolyzing human or non-human animal blood to extract the HbF. The hemolysis may be performed by, for example, adding a buffer solution to a precipitate obtained by centrifugation of whole blood obtained by blood drawing, and freezing and thawing the resultant. The HbF solution may also be obtained by centrifuging the solution obtained by hemolysis as described above, and recovering the supernatant. The HbF solution may also be obtained by further purifying the supernatant recovered as described above by dialysis or the like.

The HbF solution preferably contains a buffer solution for pH adjustment. Examples of the buffer solution include a Tris buffer solution (Tris-HCl etc.), a carbonate buffer solution, a HEPES buffer solution, and a phosphate buffer solution.

The HbF solution may contain a stabilizer. Examples of the stabilizer include a sugar, such as sucrose or trehalose.

The ratio between the amounts of HbA2 and HbF in the HbA2/HbF solution can be appropriately selected depending on the objective. For example, the ratio between the amounts of HbA2 and HbF (HbA2: HbF) may be from 1:10 to 10:1, or may be from 1:5 to 5:1.

### <Method of Producing Hb Precision Control Substance>

A method of producing an Hb precision control substance according to an embodiment of the present disclosure includes contacting a solution containing Hb and having a pH of from 7.8 to 9.5 with an anion exchanger. A precision control substance is a sample having a known composition, which is used for accuracy management of analyses, and the precision control substance in this embodiment refers to a sample in which the composition of HbA2 and optionally HbF (that is, the amounts contained in the precision control substance) is known. Any description in the section "Method of Producing Solution Containing HbA2" can apply to the details of the steps in the method of producing an Hb precision control substance in this embodiment. Here, the terms "solution containing HbA2" and "HbA2 solution" obtained are replaced with "Hb precision control substance".

In an aspect, the method of producing an Hb precision control substance may further include recovering the solution after the contacting with the anion exchanger. The manner of the recovery of the solution is as described in the section "Method of Producing Solution Containing HbA2" above.

The pH of the Hb solution to be contacted with the anion exchanger is from 7.8 to 9.5. From the viewpoint of suppressing the binding of the HbA2 to the anion exchanger and increasing the amount of HbA2 to be recovered, the pH of the Hb solution is preferably 9.0 or less. From the viewpoint of increasing the proportion of HbA2 in the resulting precision control substance, the pH of the Hb solution is preferably 8.0 or more, more preferably 8.3 or more, still more preferably 8.5 or more, and particularly preferably 8.8 or more From these viewpoints, the pH of the Hb solution is preferably from 8.0 to 9.0, more preferably from 8.3 to 9.0, still more preferably from 8.5 to 9.0, and particularly preferably from 8.8 to 9.0.

In an aspect, the solution after the contacting with the anion exchanger has a higher proportion of HbA2 with respect to the total amount of Hb as compared with the Hb solution before the contacting with the anion exchanger.

In an aspect, the Hb solution contains a buffer solution and Hb derived from blood of a human subject that has no Hb abnormality. The detail of the aspect is as described in the section "Method of Producing Solution Containing HbA2" above.

In an aspect, the method of producing an Hb precision control substance includes mixing the solution obtained by the contacting with the anion exchanger, and a solution containing HbF. The details of this aspect are as described in the section "Method of Producing Solution Containing HbA2 and HbF" above. Here, the term "a solution containing HbA2 and HbF" or "HbA2/HbF solution" obtained is replaced with the term "Hb precision control substance".

### Examples

Next, embodiments of the present disclosure are specifically described with reference to Examples. However, embodiments of the present disclosure are not limited to these Examples.

### <Example 1: Preparation of Precision Control Substance>

### 1. Preparation of a high-concentration HbA2 solution

Peripheral blood collected from healthy humans was used as human whole blood.

### [Hemolysis]

(1) The human whole blood was centrifuged (4°C, 10000 × g, 10 minutes), and the supernatant was removed.
(2) The precipitate was suspended with the same amount of saline as the human whole blood.
(3) The suspension obtained by the procedure (2) was centrifuged (4°C, 10000 × g, 10 minutes), and the supernatant was removed.
(4) The procedures (2) and (3) were repeated.
(5) The precipitate was suspended with 20 mM EDTA-NaOH (pH 7.0) that is 1.3 times the amount of the human whole blood, and frozen and thawed. The resultant is referred to as "crude Hb solution".

### [Purification of Crude Hb Solution]

(1) Ammonium sulfate was added to the crude Hb solution such that the final concentration was 20% by mass. After confirming that the ammonium sulfate was completely dissolved, the solution was stirred at ambient temperature for 10 minutes.
(2) The solution was centrifuged (4°C, 10000 × g, 10 minutes), and the supernatant was recovered.
(3) The recovered supernatant was placed in a dialysis membrane having a molecular weight cut-off of 3500, and dialyzed with purified water as an external fluid. The external fluid was used in an amount of 10 times or more the amount of the internal fluid, and was exchanged five times at an interval of 3 hours or more.
(4) The internal fluid was centrifuged (4°C, 18600 × g, 10 minutes), and the supernatant was recovered. The resultant is referred to as "solution A".

### [Ion Exchange of Solution A]

(1) A strong anion exchange resin (Macro-PrepHighQ: Bio-Rad Laboratories, Inc.) was equilibrated with 20 mM Tris-HCl (pH 8.8).
(2) Tris-HCl (pH 8.8) was added to the solution A such that the final concentration was 20 mM. The resultant is referred to as "solution B".
(3) The solution B was mixed with an equal amount of the anion exchange resin, and the solution was recovered.
(4) 20 mM Tris-HCl (pH 8.8) in an amount equal to the anion exchange resin after the recovery of the solution in the procedure (3) was added to the anion exchange resin and mixed, and the solution was recovered.
(5) 20 mM Tris-HCl (pH 8.8) containing 150 mM NaCl in an amount equal to the anion exchange resin after the recovery of the solution in the procedure (4) was added to the anion exchange resin, and the solution was recovered.
(6) The solutions recovered in the procedures (3), (4), and (5) were mixed at a certain proportion to adjust the content of HbA2. The resultant is referred to as "solution C".

### [Enrichment of Solution C]

(1) Ammonium sulfate was added to the solution C. After confirming that the ammonium sulfate was completely dissolved, the resulting solution was stirred at ambient temperature for 10 minutes. The added amount was 5.18 g with respect to 10 mL of the solution C.
(2) The resultant was centrifuged (4°C, 10000 × g, 20 minutes), and the supernatant was removed.
(3) 37.5 mM Tris-HCl (pH 8.8) was added to the precipitate and suspended. The resultant is referred to as "solution D".

### [Dialysis of Solution D]

(1) The solution D was placed in a dialysis membrane having a molecular weight cut-off of 3500, and dialyzed with 37.5 mM Tris-HCl (pH 8.8) as an external fluid. The external fluid was used in an amount of 30 times or more the amount of the internal fluid, and was exchanged twice at an interval of 24 hours or more.
(2) The dialyzed internal fluid was centrifuged (4°C, 40000 × g, 10 minutes), and the supernatant was recovered.

### 2. Preparation of a low-concentration HbA2 solution

(1) A crude Hb solution was obtained by a hemolysis treatment performed in the same manner as in "1. Preparation of a high-concentration HbA2 solution".
(2) Ammonium sulfate was added to the crude Hb solution. After confirming that the ammonium sulfate was completely dissolved, the resulting solution was stirred at ambient temperature for 10 minutes.
(3) The resultant was centrifuged, and the supernatant was recovered.
(4) The recovered supernatant was placed in a dialysis membrane having a molecular weight cut-off of 3500, and dialyzed with purified water as an external fluid.
(5) The internal fluid was centrifuged, and the supernatant was recovered. The resultant is referred to as "solution A".
(6) Tris-HCl (pH 8.8) was added to the solution A such that the final concentration was 37.5 mM.

### 3. Preparation of an HbF solution

(1) A hemolysis treatment of human umbilical whole blood was performed in the same manner as in "1. Preparation of a high-concentration HbA2 solution" to obtain a crude Hb solution.
(2) Ammonium sulfate was added to the crude Hb solution. After confirming that the ammonium sulfate was completely dissolved, the resulting solution was stirred at ambient temperature for 10 minutes.
(3) The resultant was centrifuged, and the supernatant was recovered.
(4) The recovered supernatant was placed in a dialysis membrane having a molecular weight cut-off of 3500, and dialyzed with purified water as an external fluid.
(5) The internal fluid was centrifuged, and the supernatant was recovered. The resultant is referred to as "solution A".
(6) Tris-HCl (pH 8.8) was added to the solution A such that the final concentration was 37.5 mM.

### 4. Preparation of precision control substances

(1) The low-concentration HbA2 solution was mixed with the HbF solution, and the high-concentration HbA2 solution was mixed with the HbF solution, thereby preparing HbA2/HbF solutions having two different concentrations.
(2) A 50% (w/v) sucrose solution was added to each of the HbA2/HbF solutions having respective concentrations such that the final concentration was 8.65% (w/v).
(3) The resulting precision control substance was freeze-dried.

The precision control substance prepared from the low-concentration HbA2 solution and the HbF solution is referred to as Lv1, and the precision control substance prepared from the high-concentration HbA2 solution and the HbF solution is referred to as Lv2.

### 5. Evaluation of the precision control substances

Three lots of the precision control substances having respective concentrations were prepared (Lots 1 to 3). Using a high-performance liquid chromatography apparatus ADAMS HA-8180T (manufactured by ARKRAY Inc.), the proportions (%) of HbA2 and HbF with respect to the total Hb in the precision control substances prepared by the above method were measured. The raw materials, and the proportions (%) of HbA2 and HbF with respect to the total Hb in the precision control substances prepared are shown in Table 1.

**[Table 1]**

| | | HbA2(%) | HbF(%) |
|---|---|---|---|
| Raw material | Human peripheral blood | 2.26 | 0.77 |
| | Human umbilical whole blood | 0.00 | 84.04 |
| Precision control substance | Lot1-Lv1 | 2.62 | 5.87 |
| | Lot1-Lv2 | 6.17 | 1.77 |
| | Lot2-Lv1 | 2.61 | 5.79 |
| | Lot2-Lv2 | 6.18 | 1.79 |
| | Lot3-Lv1 | 2.62 | 5.70 |
| | Lot3-Lv2 | 6.18 | 1.78 |

### 6. Discussion

Three lots of precision control substances were prepared using healthy adult human blood, in all of which HbA2 was enriched. It was revealed that the use of this method allows for the preparation of an HbA2 precision control substance stably from normal blood without using blood from β-thalassemia patients, which is rarely available.

### <Example 2: Examination of pH in Ion Exchange>

### 1. Experimental procedure

### [Preparation]

(1) 20 mM Tris-HCl buffer solutions having pHs of 8.5, 8.8, and 9.0 were prepared.
(2) An ion exchange resin (Macro-PrepHighQ: Bio-Rad Laboratories, Inc.) was equilibrated with each of the buffer solutions.

### [Preparation of Hb Solution]

Peripheral blood collected from healthy humans was used as human whole blood.
(1) The human whole blood was centrifuged (4°C, 10000 × g, 10 minutes), and the supernatant was removed.
(2) The precipitate was suspended with the same amount of saline as the human whole blood.
(3) The suspension obtained in the procedure (2) was centrifuged (4°C, 10000 × g, 10 minutes), and the supernatant was removed.
(4) The procedures (2) and (3) were repeated again.
(5) The precipitate was suspended with 20 mM EDTA (pH 7.0) that is 1.3 times the amount of the human whole blood, and frozen and thawed.
(6) The resultant was centrifuged (4°C, 40000 × g, 10 minutes), and the supernatant was recovered. The resultant is referred to as "crude Hb solution".
(7) The Hb solution was placed in a dialysis membrane having a molecular weight cut-off of 3.500, and dialyzed with purified water as an external fluid.
(8) 0.5 M Tris-HCl was added to the dialyzed Hb solution such that the final concentration was 20 mM.
(9) 20 mM Tris-HCl was added thereto to dilute the solution such that the solution has a concentration of about one-quarter of that of the human whole blood. The resultant is referred to as "Hb specimen". Three Hb specimens having different pHs were prepared.

### [Ion Exchange]

(1) Equilibrated Macro-prep HighQ and an Hb specimen were mixed in equal amounts.
(2) The solution was recovered. The resultant is referred to as "supernatant 1".
(3) 20 mM Tris-HCl that is the same volume as that of the Hb specimen of (1) was added to the remaining resin, and the solution was suspended.
(4) The solution was recovered. The resultant is referred to as "supernatant 2".
(5) The proportion (%) of HbA2 with respect to the total Hb in each of the supernatants 1 and 2 was measured.

### 2. Results

The proportion (%) of HbA2 with respect to the total Hb in each of the supernatants 1 and 2 is shown in Table 2.

**[Table 2]**

| pH of buffer solution | Before ion exchange | Supernatant 1 | Supernatant 2 | Supernatants 1 +2 |
|---|---|---|---|---|
| 8.5 | 2.96 | 5.60 | 8.43 | 6.27 |
| 8.8 | 2.90 | 7.40 | 16.90 | 8.59 |
| 9.0 | 2.94 | 12.44 | 26.70 | 14.24 |

### 3. Discussion

It has been revealed in this examination that the proportion (%) of HbA2 in the unbound fractions in the ion-exchange (supernatants 1 and 2) is increased in a pH-dependent manner. It is believed that hemoglobins differ in isoelectric points depending on the types thereof and vary in their binding abilities to the ion exchange resin in a pH-dependent manner, and that, therefore, hemoglobins other than HbA2 are preferentially bound to the ion exchange resin under specific conditions, whereby the proportion of HbA2 in the unbound fractions is relatively higher.

## Claims

1. A method of producing a solution containing hemoglobin A2, the method comprising contacting a solution containing hemoglobin and having a pH of from 7.8 to 9.5 with an anion exchanger.

2. The method of producing a solution containing hemoglobin A2 according to claim 1, further comprising recovering the solution after the contacting with the anion exchanger.

3. The method of producing a solution containing hemoglobin A2 according to claim 1 or 2, wherein the solution containing hemoglobin has a pH of from 8.0 to 9.0.

4. The method of producing a solution containing hemoglobin A2 according to any one of claims 1 to 3, wherein the solution after the contacting with the anion exchanger has a higher proportion of hemoglobin A2 with respect to a total amount of hemoglobin as compared with the solution before the contacting with the anion exchanger.

5. The method of producing a solution containing hemoglobin A2 according to any one of claims 1 to 4, wherein the solution containing hemoglobin and having a pH of from 7.8 to 9.5 contains a buffer solution and hemoglobin derived from blood of a human subject that has no hemoglobin abnormality.

6. A method of producing a solution containing hemoglobin A2 and hemoglobin F, the method comprising mixing a solution containing hemoglobin A2, obtained by the production method according to any one of claims 1 to 5, and a solution containing hemoglobin F.

7. A method of producing a hemoglobin precision control substance, the method comprising contacting a solution containing hemoglobin and having a pH of from 7.8 to 9.5 with an anion exchanger.

8. The method of producing a hemoglobin precision control substance according to claim 7, further comprising recovering the solution after the contacting with the anion exchanger.

9. The method of producing a hemoglobin precision control substance according to claim 7 or 8, wherein the solution containing hemoglobin has a pH of from 8.0 to 9.0.

10. The method of producing a hemoglobin precision control substance according to any one of claims 7 to 9, wherein the solution after the contacting with the anion exchanger has a higher proportion of hemoglobin A2 with respect to a total amount of hemoglobin as compared with the solution before the contacting with the anion exchanger.

11. The method of producing a hemoglobin precision control substance according to any one of claims 7 to 10, wherein the solution containing hemoglobin and having a pH of from 7.8 to 9.5 contains a buffer solution and hemoglobin derived from blood of a human subject that has no hemoglobin abnormality.

12. The method of producing a hemoglobin precision control substance according to any one of claims 7 to 11, the method comprising mixing the solution obtained by the contacting with the anion exchanger, and a solution containing hemoglobin F.
